# EUROPÄISCHE PATENTANMELDUNG

(11) **EP 2 759 314 A1**
(43) Veröffentlichungstag der Anmeldung: **30.07.2014**
(21) Anmeldenummer: 14150881.2
(22) Anmeldetag: 13.01.2014
(51) Int. Cl.: A61N 1/36, A61B 18/14, A61N 1/05, A61B 18/00

(54) **Katheter zur Modulation der renalen Nerven**

(30) Priorität: 28.01.2013 US 201361757238 P
(71) Anmelder: Biotronik AG, 8180 Bülach (CH)
(72) Erfinder: Degen, Nicolas, 8222 Beringen (CH)
(74) Vertreter: Lindner-Vogt, Karin L.

(57) **Zusammenfassung**

Die Erfindung betrifft einen Katheter mit einem Schaft (1) und einer distalen Spitze (3). Um eine z.B. renale Ablation zu vereinfachen einer kürzere Schmerzexposition auf Seiten des Patienten zu erreichen, ist zwischen dem Schaft (1) und der Spitze (3) ein torsionssteifer, um seine Achse drehbarer Draht (5) angeordnet, der mit einer um den Draht (5) aufgerollten, vorgespannten Folie (7, 7.1, 7.2, 7.3, 7.4) verbunden ist, auf deren außen liegender Oberfläche (8) mindestens ein Leiterpunkt (9), vorzugsweise eine Vielzahl von Leiterpunkten, angeordnet ist, wobei jeder Leiterpunkt (9) mit einer Strom- und/oder Spannungsquelle verbindbar ist.

## Beschreibung

Die vorliegende Erfindung betrifft einen Katheter mit einem Schaft und einer distalen Spitze.

Als Katheter werden im Allgemeinen Röhrchen oder Schläuche unterschiedlichen Durchmessers aus verschiedenen Materialien bezeichnet, mit denen Hohlorgane wie Harnblase, Magen, Darm, Blutgefäße oder das Herz sondiert, entleert, gefüllt, gespült oder anderweitig minimalinvasiv behandelt werden können. Der in den zu behandelnden Körper eines Menschen oder Tiers einzuführende rohr- oder schlauchförmige Abschnitt des Katheters wird als Schaft bezeichnet, an dessen distalem Ende die Katheterspitze angeordnet ist, welche von den Katheterelementen am weitesten in den zu behandelnden Körper eindringt.

Es sind bereist intravaskuläre Katheter, vor allem für die Anwendung im Herz-Thoraxbereich, mit einer aktiven oder passiven Elektrode bekannt, welche in Hauptvenen oder -arterien, beispielsweise in die Vena Femoralis, eingeführt und von dort aus an unterschiedliche Stellen im Herzen bzw. zu den Herzkranzgefäßen vorgeschoben werden können. Diese Katheter werden dafür verwendet, die elektrische Aktivität des Herzens darzustellen oder zu stimulieren oder Bereiche mit abnormaler elektrischer Aktivität abzutragen. Die zuletzt genannte Ablationstherapie dient z.B. als Therapie gegen Herzrhythmusstörungen.

Ferner werden heutzutage Katheter z.B. dafür eingesetzt, um mittels Neuromodulation der renalen Nerven (plexus renalis) eine Blutdrucksenkung zu erreichen. Diese Therapie beruht darauf, dass der an der renalen Arterie anliegende sympathische Nervenstrang einen Einfluss auf den Blutdruck hat. In diesem Zusammenhang ist bekannt, dass eine teilweise Traumatisierung dieses Nervs eine anhaltende Blutdruckreduktion bewirkt und dass diese Behandlung insbesondere bei therapierefraktären Patienten, bei denen konventionelle pharmakologische Ansätze nicht oder nicht mehr anschlagen, erfolgversprechend ist.

Für eine derartige Neuromodulation des Nervs wird derzeit ein sogenanntes Single-Pole-System mit einer einzigen Elektrode zur Ablation eingesetzt. Für jeden Behandlungspunkt muss das System vom Arzt neu ausgerichtet werden. Jede einzelne Ablation an dem Nerv ist dabei für den Patienten schmerzhaft, so dass das sequentielle Vorgehen mit dem Single-Pole-System für den Patienten und den Arzt häufig als unangenehm empfunden wird. Ein Nachteil dieses Systems besteht ferner darin, dass ein großer Teil der Leistung der Elektrode nicht thermisch im Zielgewebe umgesetzt sondern durch das die Elektrode umfließende Blut aufgenommen wird. Durch die Leistungsabgabe ins Blut kann zudem eine unerwünschte Koagulation des Blutes im Bereich der Ablationsstelle auftreten.

Aus der Druckschrift US 5,239,999 ist ein endokardiales Aufnahme- und/oder Ablationssystem zum Einführen in eine Herzkammer bekannt, bei dem an einem Katheterschaft ein flexibles Band vorgesehen ist, das in einem ersten Zustand spiralförmig um ein Rohr herum gewickelt angeordnet ist. Auf der Oberfläche des Bandes ist eine Vielzahl von Elektroden vorgesehen. Durch eine Drehung und Verschiebung des Rohrs in Längsrichtung des Katheters wird der Katheter in einen zweiten Zustand überführt, in dem das Band mit seiner äußeren Oberfläche an dem zu behandelnden Gewebe eines Gefäßes oder Körperhohlraums angelegt wird. Durch die kombinierte Rotations- und Translationsbewegung des Katheterrohrs wird die von dem Band gebildete Spirale aufgeweitet. Der Nachteil dieser Lösung besteht in der kombinierten Rotations- und Translationsbewegung des Rohrs, welche bei der Handhabung des Katheters häufig Schwierigkeiten bereitet. Zudem sind die Anlegekräfte bei der bekannten Lösung ungleichmäßig über die Länge des Bandes verteilt, so dass insbesondere die Endabschnitte des Bandes und somit einige Elektroden nicht sicher an dem zu behandelnden Gewebe anliegen.

Die Aufgabe der vorliegenden Erfindung besteht demnach darin, einen Katheter zu schaffen, mit dem eine einfachere Ablation der renalen Nerven an mehreren Punkten gleichzeitig erreicht wird. Somit wird die Gesamtbehandlungszeit reduziert und der Patient über einen kürzeren Zeitraum einem prozeduralen Schmerz ausgesetzt.

Die obige Aufgabe wird durch den in Anspruch 1 angegebenen Gegenstand gelöst. Insbesondere weist der erfindungsgemäße Katheter zwischen dessen Schaft und distaler Spitze einen torsionssteifen, um seine Achse drehbaren Draht auf, der mit einer um den Draht aufgerollten, vorgespannten flächigen Folie verbunden ist, auf deren außen liegender Oberfläche mindestens ein Leiterpunkt, vorzugsweise eine Vielzahl von Leiterpunkten, angeordnet ist, wobei jeder Leiterpunkt mit einer Strom- und/oder Spannungsquelle verbindbar ist.

Der Vorteil der erfindungsgemäßen Lösung besteht darin, dass auf der flächigen Folie insbesondere eine Vielzahl von Leiterpunkten (Elektroden) gewissermaßen in einem zweidimensionalen Muster angeordnet werden können, welche ein gleichzeitiges oder ein sequentielles Abladieren an mehreren Punkten des renalen Nervs ermöglichen. Hierfür ist keine Änderung der Position oder lediglich eine geringere Anzahl von Repositionierungsschritten erforderlich. Die Behandlungszeit und die Schmerzexposition des Patienten werden dadurch verkürzt.

Zur Behandlung des jeweiligen Gewebes eines Gefäßes oder sonstigen Körperhohlraums wird die aufgerollte und durch das Aufrollen vorgespannte flächige Folie durch Drehung des Drahtes um seine Achse abgerollt. Die Folie wird hierdurch entspannt und liegt dann gleichmäßig an der Wand des zu behandelnden Gefäßes an. Dadurch wird jeder einzelne, an der Oberfläche der Folie liegende elektrisch leitfähige Leiterpunkt unmittelbar und direkt an die inneren Oberfläche des Gefäßes oder sonstigen Körperhohlraums angelegt, so dass ein elektrischer Kontakt direkt mit dem Gewebe gegeben ist. Die Folie schmiegt sich gewissermaßen an das Gewebe an, da sie analog zu einer Spiralfeder das Bedürfnis hat, in den entrollten, entspannten Zustand überzugehen. Gegen den Blutstrom sind die an dem Gewebe anliegenden Leiterpunkte dabei weitestgehend galvanisch isoliert, so dass kein Strom in das Blut abfließt. Hierdurch wird ermöglicht, für das Veröden eine geringere Stromstärke bzw. eine geringere Leistung zu verwenden, so dass potentiell die Gefahr von Gewebe-Sekundärverbrennungen geringer wird.

Die zweidimensionale Anordnung der Leiterpunkte auf der Folie in Form eines Musters gibt die räumliche Verteilung der Ablationspole vor. Die Leiterpunkte können über die Verbindung mit der Strom- und/oder Spannungsquelle mit einem hochfrequenten Strom zur Verödung der Nerven nahe der Gefäßwand versorgt werden. Während dieser Prozedur werden die Leiterpunkte von dem auf der Rückseite (innere Oberfläche) der Folie entlang strömenden Blut gekühlt. Das System arbeitet in vorteilhafter Weise ohne Unterbrechung des physiologischen Blutflusses.

In einer besonders bevorzugten Ausführungsform des erfindungsgemäßen Katheters ist zusätzlich eine Hülse mit einem Schlitz vorgesehen, in der der Draht angeordnet und die aufgerollte Folie aufnehmbar ist, wobei die Folie durch den Schlitz hindurch abrollbar ist. Die Hülse dient dem Schutz der Folie und der darauf angeordneten Leiterpunkte während des Einführens des Katheters bis zu der zu behandelnden Stelle im Körper. Vorzugsweise ist die Folie in dem Zustand mit aufgerollter Folie derart in der Hülse angeordnet, dass der am weitesten von der Verbindungsstelle mit dem Draht entfernten Abschnitt der Folie außen auf der Oberfläche der Hülse aufliegt und diese zumindest teilweise oder vollständig bedeckt. Durch die Befestigung der Folie an einer außen liegenden Kante des Schlitzes der Hülse wird ein sehr gutes Aufspannverhalten der Folie erreicht.

Für eine lokale Temperaturmessung sind außen an der außen liegenden Oberfläche der Folie mindestens ein Thermoelement und/oder ein thermoresistiver Widerstand angeordnet.

In einer Weiterbildung der Erfindung ist die Folie und/oder die Hülse in eine Richtung quer zur Längsrichtung des Katheters lediglich geschlitzt und/oder in mindestens zwei Abschnitte unterteilt und mit mindestens einem quer zur Längsrichtung des Katheters verlaufenden, insbesondere hinsichtlich Biegen und/oder Druck und/oder Zug flexiblen, vorzugsweise elastischen Segment versehen. Die Folie und/oder die Hülse bestehen in der zuletzt genannten Variante dieses Ausführungsbeispiels aus mehreren Abschnitten, welche durch die elastischen Segmente verbunden sind. Ferner kann auch ein derartiges elastisches Segment zwischen der Spitze (oder dem Schaft) und der Folie (oder dem Folienabschnitt) und/oder der Hülse (oder dem Hülsenabschnitt) angeordnet sein. Hierdurch kann die Biegesteifigkeit der Konstruktion verringert werden, so dass eine Seitwärtsbewegung des Katheters zugelassen wird.

In einem bevorzugten Ausführungsbeispiel der Erfindung enthält die Folie zumindest abschnittsweise ein flexibles Material, vorzugsweise ein Polyimid und/oder ein LCP (Flüssigkristallpolymer).

Nach der vorliegenden Erfindung dient das Folienmaterial als Trägermaterial für Leiterstrukturen auf ihrer äußeren Oberfläche. Hierbei sind die Leiterpunkte durch Leiterbahnen mit einem entsprechenden, an der Leiterfolie angeordneten Anschluss verbunden, der wiederum mit einer Spannungs- oder Stromquelle in elektrischem Kontakt steht. Insbesondere ist für die Folie ein elastisches Material geeignet, das eine elastische Verformung langfristig realisiert und selbst bei Temperaturen von über 50°C nur ein geringes viskoelastisches Verhalten aufweist.

Die Folie weist erfindungsgemäß vorzugsweise die Form eines Rechtecks auf und ist blattförmig ausgebildet, wobei die Folie besonders bevorzugt an einer Seitenkante mit dem Draht verbunden ist. Die ggf. mehrschichtige Folie kann alternativ eine andere blattförmige Form (z.B. Dreieck oder Halbkreis) aufweisen, die je nach Einsatzzweck (z.B. entsprechend der Form des zu behandelnden Organs) variiert werden kann. Das Material der Folie ist derart ausgewählt, dass es biegeelastisch ist und einer Verbiegung eine Gegenspannung entgegensetzt, so dass die Folie beim Aufrollen auf den Draht gespannt wird. Demgegenüber entspannt sich die Folie beim Abrollen.

Erfindungsgemäß ist unter dem Begriff "Aufrollen" der Folie gemeint, dass ein erster Abschnitt der Folie durch eine Rotationsbewegung innerhalb eines weiteren Abschnitts der Folie angeordnet wird.

In einem weiteren bevorzugten Ausführungsbeispiel ist der Draht mit einem Kardanantrieb verbunden, der eine Drehung des Drahts um seine Achse zum Auf- und Abrollen der Folie bewirkt.

In einem weiteren Ausführungsbeispiel kann die Folie an ihrer inneren Oberfläche ein gut wärmeleitendes Material aufweisen, um eine Kühlung der Folie z.B. durch Blut oder durch ein von außen administriertes Kühlmittel (z.B. niedrig temperierte Ringerlösung) oder durch eine andere Körperflüssigkeit zu begünstigen. Geeignete wärmeleitende Materialien können von der Elektrode galvanisch entkoppelte Edelmetallbeschichtungen sein, welche auch die erforderliche Biokompatibilität aufweisen.

Der erfindungsgemäße Katheter mit der flexiblen Folie und dem mindestens einen Leiterpunkt an seiner außen liegenden Oberfläche lässt sich als Plattform für weitere vaskuläre, kardiale, viszerale oder neuronale Anwendungen verwenden. Hierfür können eine beliebige Anzahl von Sensoren sowie aktive oder passive Elektroden auf der Oberfläche der Folie angeordnet werden. Die Anwendungsmöglichkeiten betreffen zum Beispiel EKG-Anleitungen für die Speiseröhre oder die Luftröhre, Temperaturmessungen in der Speiseröhre bei einer Vorhof-Ablation, neuronale Denervierungen in weiteren Organen, Stimulationen, zum Beispiel der Barorezeptoren in der Carotis, oder Messungen neuronaler Aktivitäten (Chemo-, Barorezeptoren, MSNA (Muscle Sympathetic Nerve Activity)).

Es ist von Vorteil, dass bei einer Anwendung des erfindungsgemäßen Katheters in Gefäßen jeweils das dort enthaltene Fluid (Blut oder Luft) weiterhin durch das jeweilige Gefäß fließen kann.

Gegenüber dem Stand der Technik nach der Druckschrift US 5,239,999 liegt das flexible, die Elektroden aufweisende Element bei der vorliegenden Erfindung im entspannten Zustand an dem zu behandelnden Gewebe an, so dass sich alle Elektroden gleichermaßen und direkt an das Gewebe anschmiegen. Zudem erfolgt das Auf- und Abrollen der Folie nach der vorliegenden Erfindung mit einer ausschließlich rotatorischen Bewegung. Für das Anlegen der Elektroden an das Gewebe ist daher keine translatorische Bewegung des Katheters notwendig.

Weitere Ziele, Merkmale, Vorteile und Anwendungsmöglichkeiten der Erfindung ergeben sich aus der nachfolgenden Beschreibung von Ausführungsbeispielen eines erfindungsgemäßen Katheters anhand der Figuren. Dabei bilden alle beschriebenen und/oder bildlich dargestellten Merkmale für sich oder in beliebiger Kombination den Gegenstand der vorliegenden Erfindung, unabhängig von ihrer Zusammenfassung in den einzelnen Ansprüchen oder deren Rückbeziehung.

Es zeigen schematisch:
- Fig. 1: eine Ansicht eines ersten Ausführungsbeispiels eines erfindungsgemäßen Katheters von der Seite im entspannten Zustand der Folie,
- Fig. 2: das Ausführungsbeispiel gemäß Fig. 1 im gespannten Zustand der Folie in einem Querschnitt, wobei der Katheter in einem Gefäß angeordnet ist,
- Fig. 3: das Ausführungsbeispiel gemäß Fig. 1 im entspannten Zustand der Folie in einem Querschnitt, wobei der Katheter analog zu Fig. 2 in einem Gefäß angeordnet ist,
- Fig. 4: ein zweites Ausführungsbeispiel eines erfindungsgemäßen Katheters in einer Ansicht von der Seite im entspannten Zustand und
- Fig. 5: das Ausführungsbeispiel gemäß Fig. 4, ebenfalls im entspannten Zustand, wobei der distale Abschnitt des Katheters gebogen ist.

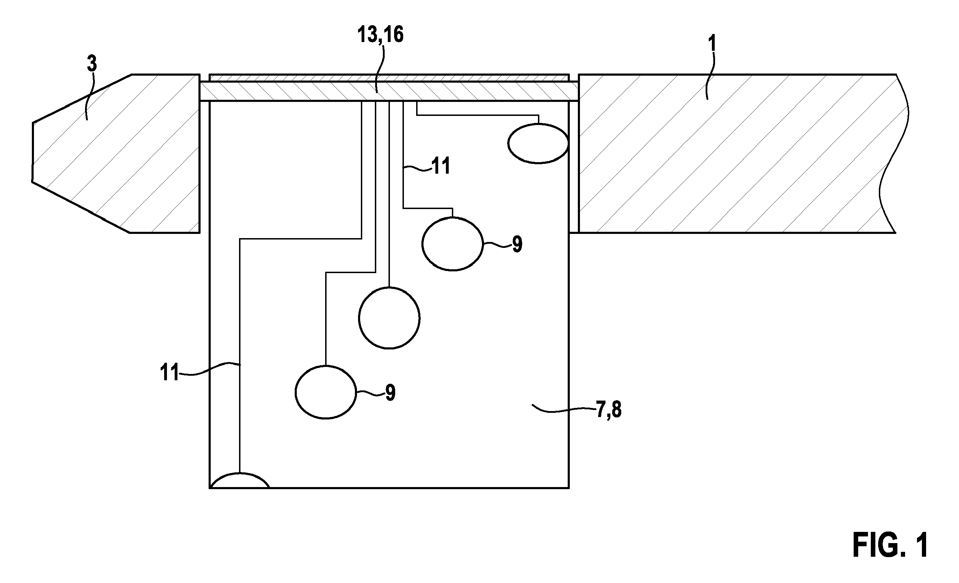

In Figur 1 ist ein erstes Ausführungsbeispiel eines erfindungsgemäßen Katheters mit einem Schaft 1 dargestellt, an dessen distaler Spitze 3 eine an einem torsionssteifen Draht 5 befestigte flexible Folie 7 angeordnet ist. Die elastische Folie 7 hat eine im Wesentlichen rechteckige, blattartige Form, wobei die Folie 7 mit einer Seitenkante an dem Draht 5 befestigt ist. Auf der äußeren Oberfläche (Außenseite) 8 der Folie 7 ist eine Vielzahl von Leiterpunkten 9 angeordnet. Jeder Leiterpunkt 9 ist über eine Leiterbahn 11 mit einer Strom- oder Spannungsquelle (nicht dargestellt) verbunden, welche vorzugsweise außerhalb des Katheters an dessen proximalen Ende angeordnet ist. In dem ersten Ausführungsbeispiel eines erfindungsgemäßen Katheters sollen die Leiterpunkte 9 insbesondere dazu dienen, mittels eines entsprechenden elektrischen Stroms Bereiche der renalen Nerven zu veröden, so dass jeder Leiterpunkt 9 auch als aktive Elektrode angesehen werden kann.

In dem in Figur 1 dargestellten ersten Ausführungsbeispiel sind die Leiterpunkte 9 in einer Diagonale über die Außenseite 8 der Folie 7 verteilt angeordnet. Jede beliebige andere Anordnung der Leiterpunkte 9 ist ebenfalls denkbar. In einem weiteren Ausführungsbeispiel können auf der Oberfläche der Folie 7 ferner mindestens ein Thermoelement, mindestens ein Sensor und/oder mindestens eine passive Elektrode angeordnet sein.

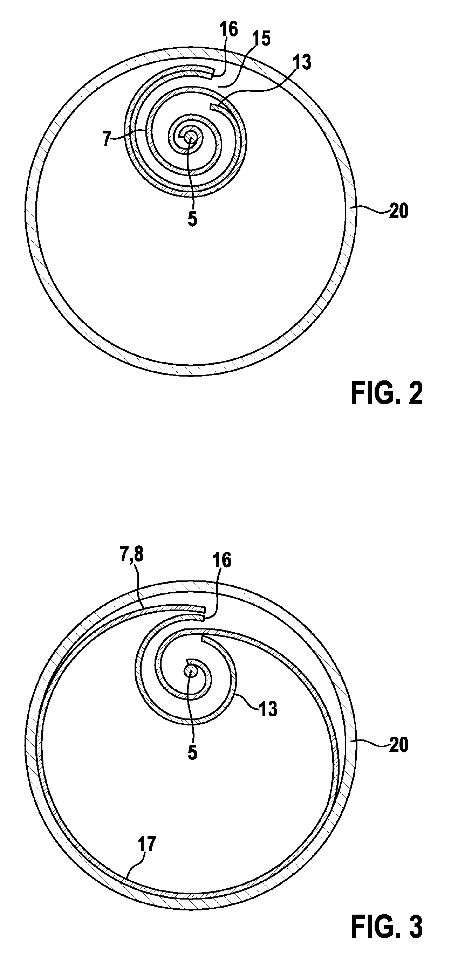

In dem in Figur 2 dargestellten gespannten Zustand der Folie 7, in dem der Katheter mit der Folie 7 einen kleinen Außendurchmesser aufweist und in dem der Katheter zu der zu behandelnden Stelle im Körper des Behandelten geführt wird, ist die flexible Folie 7 um den Draht 5 in einer rohrförmigen Hülse 13 eingerollt angeordnet, wobei der dem Draht 5 gegenüber liegende Abschnitt der Folie 7 um die Hülse 13 herum gelegt ist. Hierbei ist die Folie 7 an einer Seitenkante mit der außen liegenden Kante 16 des Schlitzes 15 der Hülse 13 befestigt. Die Folie 7 hat zusammen mit der Hülse 13 gewissermaßen die Form und Wirkungsweise einer Unruh, was ein sehr gutes Aufspannverhalten der Folie 7 zu erreichen.

Zur Behandlung, beispielsweise zur renalen Ablation der renalen Nerven wird der Draht 5 beispielsweise mittels eines am proximalen Ende des Katheters angeordneten Kardanantriebs um seine Längsachse derart gedreht, dass die Folie 7 durch einen Schlitz 15 der Hülse 13, der parallel zur Längsachse des Katheters bzw. des Drahtes 5 verläuft, abgerollt und in den entspannten Zustand überführt wird, in dem die Folie 7 an der Innenwand des Gefäßes 20 anliegt. In dem entspannten Zustand hat der Abschnitt des Katheters, in dem die Folie 7 angeordnet ist, einen gegenüber dem gespannten Zustand deutlich größeren Durchmesser. Durch den Übergang in den entspannten Zustand liegen die Leiterpunkte 9 direkt an der Gefäßwand an und die räumliche Verteilung der Leiterpunkte 9 ermöglicht eine Behandlung an mehreren Punkten der renalen Nerven gleichzeitig oder sequentiell ohne weitere Positionierung. Hierfür werden die Leiterpunkte 9 über die Leiterbahnen 11 mit einem hochfrequenten Strom versorgt, der die Nerven nahe der Gefäßwand verödet. Das durch das Gefäß laufende Blut strömt hierbei auf der Rückseite (Innenseite) 17 der Folie 7 vorbei und kühlt diese. Thermische Sensoren (nicht dargestellt), welche in der Nähe der Leiterpunkte 9 an der Außenseite 8 der Folie 7 angeordnet sein können, überwachen die Temperatur und stellen sicher, dass die maximal zulässige Temperatur im Blut nicht überschritten wird. Das System kann somit minimalinvasiv arbeiten, ohne den physiologischen Blutfluss zu unterbrechen.

Um einen besseren Wärmeübergang in das Blut zu erreichen, kann die Folie 7 auf der Innenseite 17 mit einem gut wärmeleitenden Material, beispielsweise einer Edelmetallbeschichtung versehen sein.

Da durch das dichte Anlegen der Leiterpunkte 9 an die Gefäßwand des Gefäßes 20 eine Isolation vom Blut erfolgt, treten keine Ableitströme auf. Daher ist gegenüber dem bekannten Verfahren (Single-Pole-System, d.h. ein an der Körperoberfläche angebrachten Gegenelektrode) aufgrund einer erfindungsgemäß annähernd vollständigen thermoresistiven Umsetzung der elektrisch eingebrachten Leistung an oder nahe dem therapeutisch zu behandelnden Zielgewebe eine geringere Leistung zur Ablation erforderlich. Durch das gezieltere und effizientere Einbringen der Energie in das Gewebe lässt sich die Behandlungszeit und dadurch die Dauer Schmerzexposition des Patienten verkürzen.

Ferner werden minimiert man durch die gezielte und somit besser zu steuernde Einbringung der Energie die Gefahr von Verbrennungen an der Gefäßwand.

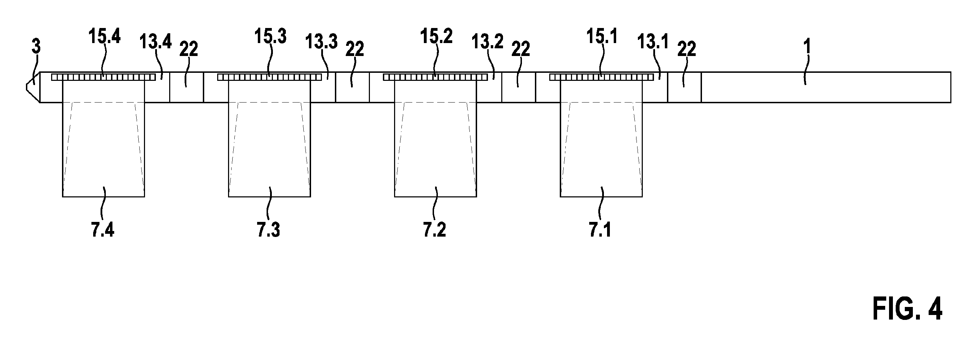

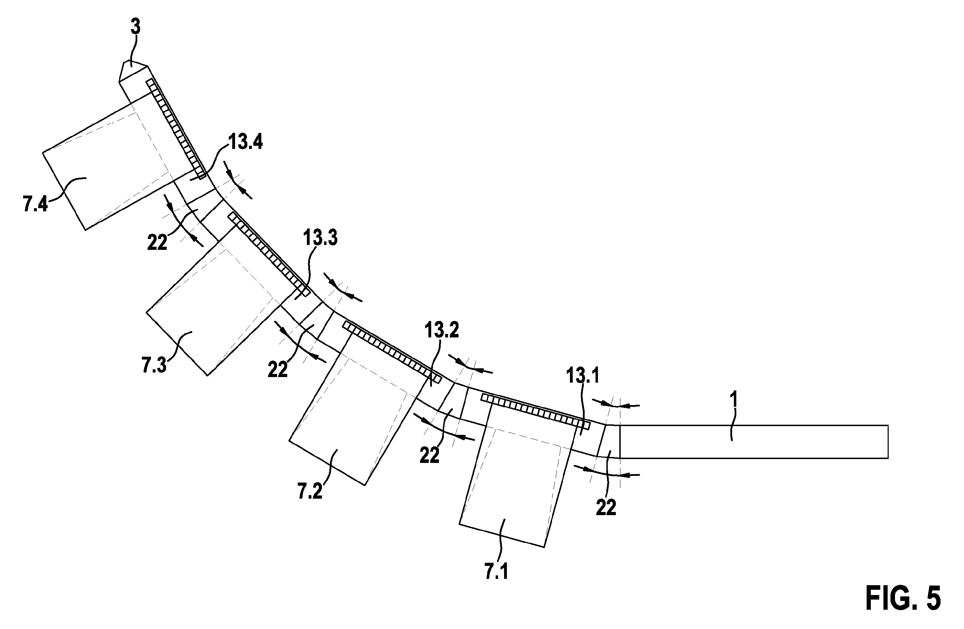

Um einen bessere Einbringung des erfindungsgemäßen Katheters durch den Körper des Behandelten bis an die renalen Nerven zu ermöglichen, ist in dem in Fig. 4 und 5 dargestellten zweiten Ausführungsbeispiel sowohl die Hülse 13 als auch die Folie 7 in insgesamt jeweils vier Folienabschnitte 7.1, 7.2, 7.3 und 7.4 bzw. Hülsenabschnitte 13.1, 13.2, 13.3 und 13.4 (jeweils mit einem Schlitz 15.1, 15.2, 15.3, 15.4) unterteilt, welche entlang der Längsrichtung des Katheters (Katheterachse) hintereinander angeordnet sind. Zwischen dem Schaft 1 und dem ersten Hülsen- und Folienabschnitt 7.1, 13.1 und den benachbarten Folien- und Hülsenabschnitten ist jeweils ein flexibles, elastisches Segment 22 vorgesehen, welches den Schaft 1 und den Hülsen- und Folienabschnitt bzw. zwei benachbarte Hülsenund Folienabschnitte insbesondere hinsichtlich Biegung flexibel miteinander verbindet.

Hierdurch kann, wie in Fig. 5 gezeigt, der erfindungsgemäße Katheter gebogen und somit durch Kurvenabschnitte im Körper des Behandelten geführt werden. Die Hülsen- und Folienabschnitte 7.1, 7.2, 7.3, 7.4 bzw. 13.1, 13.2, 13.3, 13.4 sind demgegenüber vergleichsweise biegesteif.

Jeder in Fig. 4 und 5 gezeigte Hülsen- und Folienabschnitt 7.1, 7.2, 7.3, 7.4 bzw. 13.1, 13.2, 13.3, 13.4 entspricht im Aufbau der Hülse 13 mit Folie 7 einschließlich Schlitz 15.1, 15.2, 15.3, 15.4, Draht 5 und Leiterstruktur (Leiterpunkte 9, Leiterbahnen 11), wobei die Leiterpunkte 9 selbstverständlich in einem anderen zweidimensionalen Muster angeordnet sein können.

### Bezugszeichenliste:

- 1: Schaft
- 3: Spitze, distales Ende des Katheters
- 5: Draht
- 7, 7.1, 7.2, 7.3, 7.4: Folie
- 8: Außenseite der Folie 7
- 9: Leiterpunkt
- 11: Leiterbahn
- 13, 13.1, 13.2, 13.3, 13.4: Hülse
- 15, 15.1, 15.2, 15.3, 15.4: Schlitz
- 16: außen liegende Kante des Schlitzes 15
- 17: Innenseite der Folie 7
- 20: Gefäß
- 22: flexibles Segment

## Patentansprüche

1. Katheter zwischen dessen Schaft (1) und distaler Spitze (3) ein torsionssteifer, um seine Achse drehbarer Draht (5) angeordnet ist, der mit einer um den Draht (5) aufgerollten, vorgespannten Folie (7, 7.1, 7.2, 7.3, 7.4) verbunden ist, auf deren außen liegender Oberfläche (8) mindestens ein Leiterpunkt (9), vorzugsweise eine Vielzahl von Leiterpunkten, angeordnet ist, wobei jeder Leiterpunkt (9) mit einer Strom- und/oder Spannungsquelle verbindbar ist.

2. Katheter nach Anspruch 1, **dadurch gekennzeichnet, dass** zusätzlich eine Hülse (13, 13.1, 13.2, 13.3, 13.3) mit einem Schlitz (15, 15.1, 15.2, 15.3, 15.4) vorgesehen ist, in der der Draht (5) angeordnet und die aufgerollte Folie (7, 7.1, 7.2, 7.3, 7.4) aufnehmbar ist, wobei die Folie (7, 7.1, 7.2, 7.3, 7.4) durch den Schlitz (15, 15.1, 15.2, 15.3, 15.4) hindurch abrollbar ist.

3. Katheter nach einem der vorhergehenden Ansprüche, **dadurch gekennzeichnet, dass** an der außen liegenden Oberfläche (8) der Folie (7, 7.1, 7.2, 7.3, 7.4) mindestens ein Thermoelement und/oder ein thermoresistiver Widerstand angeordnet ist.

4. Katheter nach einem der vorhergehenden Ansprüche, **dadurch gekennzeichnet, dass** die Folie (7.1, 7.2, 7.3, 7.4) und/oder die Hülse (13.1, 13.2, 13.3, 13.3) in eine Richtung quer zur Längsrichtung des Katheters geschlitzt und/oder in mindestens zwei Abschnitte unterteilt und mit mindestens einem quer zur Längsrichtung des Katheters verlaufenden flexiblen Segment (22) versehen ist.

5. Katheter nach einem der vorhergehenden Ansprüche, **dadurch gekennzeichnet, dass** die Folie (7, 7.1, 7.2, 7.3, 7.4) zumindest abschnittsweise ein flexibles Material, vorzugsweise ein Polyimid und/oder ein LCP, enthält.

6. Katheter nach einem der vorhergehenden Ansprüche, **dadurch gekennzeichnet, dass** der Draht (5) mit einem Kardanantrieb verbunden ist.
